# EUROPÄISCHE PATENTSCHRIFT

(11) **EP 2 258 285 B1**
(45) Veröffentlichungstag und Bekanntmachung des Hinweises auf die Patenterteilung: **01.05.2013**
(21) Anmeldenummer: 10005676.1
(22) Anmeldetag: 01.06.2010
(51) Int. Cl.: A61B 17/295

(54) **Medizinische Stanze**
Medical punch
Poinçonneuse médicale

(30) Priorität: 06.06.2009 DE 102009024124
(43) Veröffentlichungstag der Anmeldung: 08.12.2010
(73) Patentinhaber: Karl Storz GmbH & Co. KG, 78532 Tuttlingen (DE)
(72) Erfinder: Oberländer, Martin, 78234 Engen (DE); Sauer, Michael, 78532 Tuttlingen (DE); Bacher, Uwe, 78532 Tuttlingen (DE); Stähler, Michael, 78532 Tuttlingen (DE)
(74) Vertreter: Hofmeister, Frank

(56) Entgegenhaltungen:
- WO-A2-2007/120063
- DE-B1- 2 808 911
- DE-U1- 8 518 482

## Beschreibung

Die Erfindung betrifft eine medizinische Stanze mit einem hohlen Schaft, einer am distalen Ende des Schaftes angeordneten, aus einem starren Schneidenteil und einem gegenüber der starren Schneidteil verschiebbaren Schneidteil bestehenden Werkzeugspitze sowie mit einer am proximalen Ende des Schaftes angeordneten Handhabe, wobei der verschiebbare Schneidenteil und die Handhabe über eine im hohlen Schaft verschiebbar gelagerte Schub-/Zugeinrichtung miteinander in Wirkverbindung stehen und an der Werkzeugspitze ein die Schneidenteile in ihrer zueinander achsparallelen Ausrichtung stabilisierender Kippschutz angeordnet ist, wobei der starre Schneidenteil (7) aus einem den verschiebbaren Schneidenteil (8) umgreifenden hülsenförmigen proximalen Teil (18) und einem einstückig mit dem hülsenförmigen Teil (18) ausgebildenten distalen Stanzteil (19) besteht.

Bei medizinischen Stanzen handelt es sich um Schneidwerkzeuge mit einer starren Schneidkante und einer gegenüber der starren Schneidkante verschiebbaren Schneidkante, wobei die beiden Schneidkanten nicht wie bei einer Schere oder dergleichen um einen Drehpunkt gegeneinander verschwenkbar sind, sondern in der Regel horizontal gegeneinander verfahrbar sind.

Bei den aus der Praxis bekannten medizinischen Stanzen ist der Instrumentenschaft als Hohlschaft ausgebildet, in dem eine mit der verschiebbaren Schneidkante verbundene und über die Handhabe antreibbare Schub-/Zugeinrichtung verschiebbar gelagert ist. Diese Stanzen haben sich in der Praxis durchaus bewährt, jedoch kann es in der Praxis, insbesondere bei Stanzen, deren Schneidkanten um 45° gegenüber der Instrumentenlängsachse geneigt ausgebildet sind, zu einem Auseinanderscheren der Schneidkanten kommen, wenn die Schneidkanten in die Stanz- bzw. Schließposition verschoben werden. Dieses Ausscheren der Schneidkanten aus ihrer achsparallelen Ausrichtung zueinander verhindert ein exaktes Betätigen der medizinischen Stanze.

Um die Schneidkanten einer medizinischen Stanze achsparallel zueinander auszurichten, ist beispielsweise aus der DE 08 911C2 (Basis für den Oberbegrift des Anspruchs 1) bekannt, das verschiebbare hohle Außenrohr und die starre Innenstange über eine Nut-Feder-Verbindung relativ zueinander zu sichern. Nachteilig an dieser bekannten Konstruktion ist einerseits der hohe Fertigungsaufwand zur Ausbildung der zueinander passend zu fertigenden Verkippsicherung und andererseits die Unflexibilität bezüglich des Austausches der Schneidenteile.

Davon ausgehend liegt der Erfindung die **Aufgabe** zugrunde, eine medizinische Stanze der eingangs genannten Art so auszugestalten, dass diese bei einfachem Aufbau eine stets exakte Führung der Schneidenteile zueinander gewährleistet.

Die **Lösung** dieser Aufgabenstellung ist erfindungsgemäß dadurch gekennzeichnet, dass der starre Schneidenteil (7) und der verschiebbare Schneidenteil (8) über eine auf den hülsenförmigen Teil (18) des starren Schneidenteils (7) aufschiebbare hülsenförmige Manschette (23) zu einer Baueinheit verbindbar sind und, dass diese Baueinheit über einen am hülsenförmigen Teil (18) des starren Schneidenteils (7) ausgebildeten Kopplungsmechanismus (11) lösbar mit dem hohlen Schaft (2) verbunden ist.

Durch die erfindungsgemäße Kopplung des starren Schneidenteils mit dem hohlen Schaft über einen am hülsenförmigen Teil ausgebildeten Kopplungsmechanismus, beispielsweise in der Form einer Bajonettverbindung, wird gewährleistet, dass die erfindungsgemäße medizinische Stanze einfach und gut zu reinigen ist und darüber hinaus das Auswechseln der Schneidenteile möglich ist.

Zur Aufnahme und Führung des verschiebbaren Schneidenteils ist im hülsenförmigen Teil des starren Schneidenteils vorteilhafterweise eine Führungsbahn ausgebildet, die die Außenkontur des aufzunehmenden verschiebbaren Schneidenteils im Wesentlichen formschlüssig umschließt. Die lagegerechte Führung des verschiebbaren Schneidenteils in dieser Führungsbahn kann erfindungsgemäß dadurch stabilisiert werden, dass am verschiebbaren Schneidenteil mindestens ein Führungselement ausgebildet ist, das in eine korrespondierende Aufnahme der Führungsbahn des starren Schneidenteils eingreift.

Gemäß einer ersten Ausführungsform der Erfindung wird vorgeschlagen, dass der Kippschutz als im proximalen Bereich eines Schneidenteils ausgebildetes Widerlager, beispielsweise in Form einer wulstartigen Erhebung, ausgebildet ist, an dem der andere Schneidenteil anliegt, wodurch sich eine gegenseitige Lagestabilisierung der Schneidenteile zueinander ergibt.

Gemäß einer zweiten Ausführungsform der Erfindung wird vorgeschlagen, dass der Kippschutz als im distalen Bereich an einem Schneidenteil angeordneter Führungsstift ausgebildet ist, der in eine entsprechende Aufnahme im anderen Schneidenteil eingreift. Bei dieser Ausgestaltungsform erfolgt die gegenseitige Stabilisierung über ein im Wesentlichen formschlüssiges Ineinandergreifen von Teilbereichen der beiden Schneidenteile.

Mit einer dritten Ausführungsform der Erfindung wird vorgeschlagen, dass der Kippschutz als beide Schneidenteile im proximalen Bereich umgreifende Hülse ausgebildet ist. Diese Art der Ausbildung des Kippschutzes stellt eine besonders einfache konstruktive Maßnahme dar, da die Lagestabilisierung der Schneidenteile ohne besondere konstruktiven Anpassung der Schneidenteile über den äußeren Druckring erfolgt.

Um weiterhin zu gewährleisten, dass die von der Handhabe auf die Schub-/Zugeinrichtung ausgeübte Axialverschiebung der Schub-/Zugeinrichtung im Wesentlichen spielfrei auf den verschiebbaren Schneidenteil übertragen wird, wird erfindungsgemäß vorgeschlagen, dass der verschiebbare Schneidenteil zumindest kraftschlüssig mit der Schub-/Zugeinrichtung verbunden ist, wobei die Schub-/Zugeinrichtung über das distale Ende des Instrumentenschaftes in den Schaft einsetzbar und aus dem Schaft herausziehbar ist.

Gemäß einer bevorzugten Ausführungsform der Erfindung wird weiterhin vorgeschlagen, dass der starre Schneidenteil und der verschiebbare Schneidenteil über eine auf den hülsenförmigen Teil des starren Schneidenteils aufschiebbare hülsenformige Manschette zu einer Baueinheit verbindbar sind. Durch diese hülsenförmige Manschette wird der starre Schneidenteil unverlierbar an dem in der Führungsbahn des starren Schneidenteils gelagerten verschiebbaren Schneidenteil festgelegt, wodurch eine Montageeinheit geschaffen wird, die über den am hülsenförmigen proximalen Teil des starren Schneidenteils angeordneten Kopplungsmechanismus am hohlen lnstrumentenschaft festlegbar ist.

Die den verschiebbaren Schneidenteil und den starren Schneidenteil zu einer Baueinheit verbindende Manschette bildet gemäß einer praktischen Ausführungsform der Erfindung gleichzeitig dem die beiden Schneidenteile gegeneinander stabilisierenden, als Hülse ausgebildeten Kippschutz.

Schließlich wird mit der Erfindung vorgeschlagen, dass am starren Schneidenteil die Verschiebbarkeit des verschiebbaren Schneidenteils in die distale Richtung begrenzender Anschlag ausgebildet ist.

Weitere Merkmale und Vorteile der Erfindung ergeben sich anhand der zugehörigen Zeichnungen, in denen mehrere Ausführungsbeispiele einer erfindungsgemäßen medizinischen Stanze nur beispielhaft dargestellt sind, ohne die Erfindung auf diese Ausführungsbeispiele zu beschränken. In den Zeichnungen zeigt:
- Fig. 1: eine schematische Seitenansicht einer erfindungsgemäßen medizini- schen Stanze;
- Fig.2: eine vergrößerte Schnittdarstellung des Details II, eine Werkzeugspitze dem Stand der Technik darstellend;
- Fig. 3: eine vergrößerte Schnittdarstellung des Details III, eine erste erfin- dungsgemäße Ausführungsform einer Werkzeugspitze darstellend ;
- Fig.4: eine vergrößerte Schnittdarstellung des Details III, eine zweite erfin- dungsgemäße Ausführungsform einer Werkzeugspitze darstellend ;
- Fig. 5: eine vergrößerte Schnittdarstellung des Details III, eine dritte erfin- dungsgemäße Ausführungsform einer Werkzeugspitze darstellend ;
- Fig. 6: eine teilweise geschnittene perspektivische Ansicht einer vierten erfin- dungsgemäße Ausführungsform einer Werkzeugspitze in der geöffneten Position und
- Fig.7: einen Schnitt entlang der Linie VII-VII gemäß Fig. 6, jedoch die Werk- zeugspitze in der geschlossenen Position darstellend.

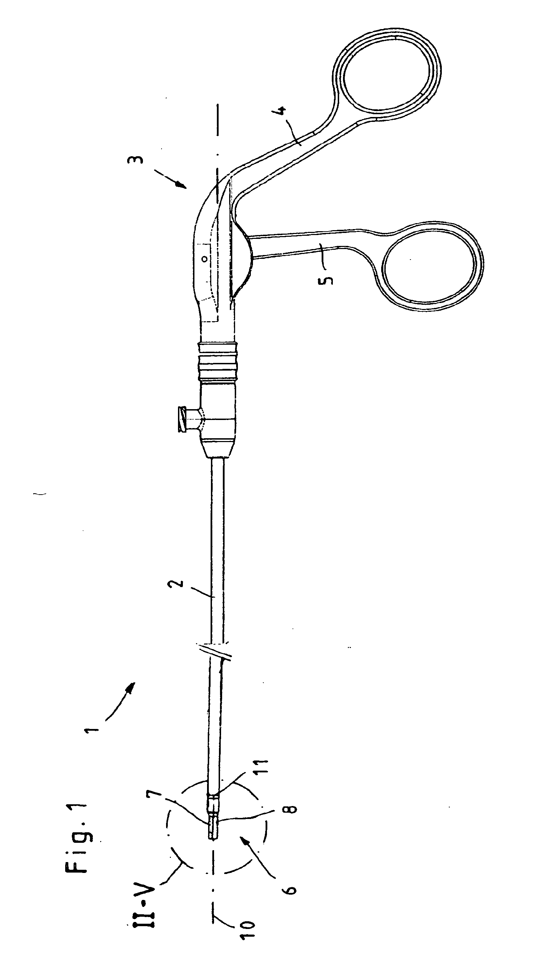

Die in der Abbildung Fig. 1 vollständig dargestellte medizinische Stanze 1 besteht im Wesentlichen aus einem als Hohlschaft ausgebildeten Schaft 2, an dessen proximalem Ende eine Handhabe 3 angeordnet ist, die aus einem starren Griffteil 4 und einem gegenüber dem starren Griffteil 4 verschwenkbaren Griffteil 5 besteht. Am distalen Ende des Schaftes 2 ist eine Werkzeugspitze 6 angeordnet, die aus einem starren Schneidenteil 7 und einem gegenüber dem starren Schneidenteil 7 verschiebbaren Schneidenteil 8 besteht, wobei der verschiebbare Schneidenteil 8 über eine verschiebbar im Schaft 2 gelagerte Schub-/Zugeinrichtung 9 mit dem verschwenkbaren Griffteil 5 der Handhabe 3 in Wirkverbindung steht.

Der verschiebbare Schneidenteil 8 der Werkzeugspitze 6 und der verschwenkbare Griffteil 5 der Handhabe 3 stehen über die Schub-/Zugeinrichtung 9 derart in Wirkverbindung miteinander, dass durch das Verstellen des verschwenkbaren Griffteils 5 der Handhabe 3 der verschiebbare Schneidenteil 8 über eine Verlagerung der Schub-/Zugeinrichtung 9 in Richtung der Längsachse 10 des Schaftes 2 auf den starren Schneidenteil 7 der Werkzeugspitze 6 zu in eine geschlossene Stanzposition bzw. umgekehrt von dem starren Schneidenteil 7 fort in eine offene Position verschiebbar ist.

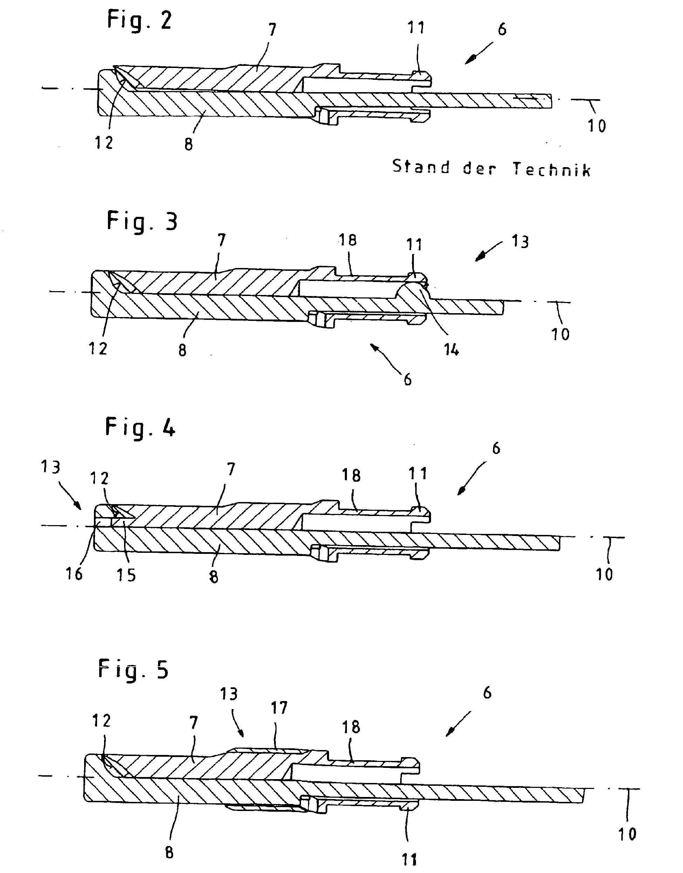

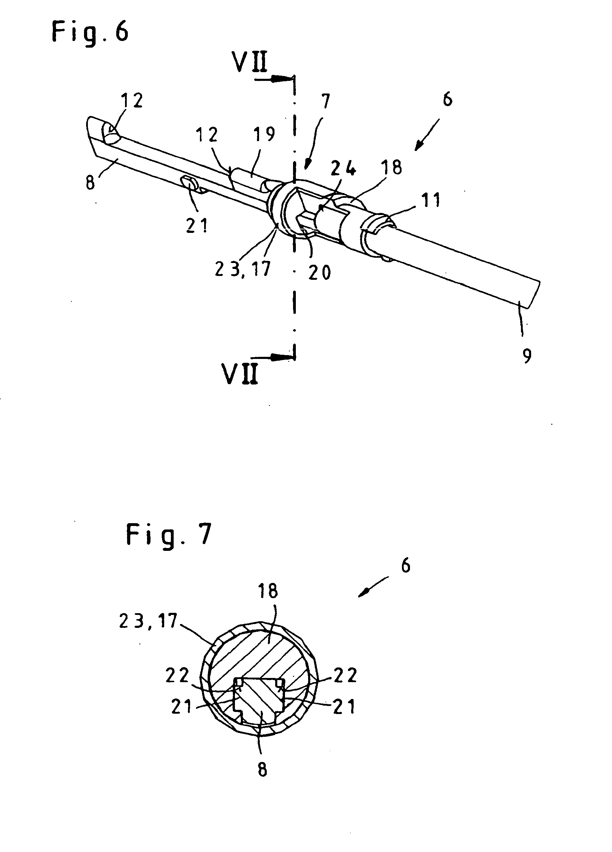

Bei der dargestellten Ausführungsform bewirkt das Zusammendrücken der Griffteile 4 und 5 der Handhabe 3 eine Verlagerung der Schub-/Zugeinrichtung 9 in die proximale Richtung und somit ein Verschieben des verschiebbaren Schneidenteils 8 in die in Fig. 2 bis 5 dargestellte geschlossene Stanzposition der Werkzeugspitze 6. Umgekehrt bewirkt das Auseinanderdrücken der Griffteile 4 und 5 der Handhabe 3 eine Verlagerung der Schub-/Zugeinrichtung 9 in die distale Richtung und somit ein Verschieben des verschiebbaren Schneidenteils 8 in die in Fig. 6 dargestellte offene Position der Werkzeugspitze 6, in der der verschiebbare Schneidenteil 8 und der starre Schneidenteil 7 am weitesten voneinander entfernt sind.

Der Aufbau der Werkzeugspitze 6 mit dem starren Schneidenteil 7 und dem verschiebbaren Schneidenteil 8 ist insbesondere den Detailansichten gemäß Fig. 2 bis Fig. 6 zu entnehmen, die vier Ausführungsbeispiele zur Ausgestaltung der Werkzeugspitze 6 zeigen. In allen Fällen bildet der verschiebbare Schneidenteil 8 das distale Ende der verschiebbar im hohlen Schaft 2 gelagerten Schub-/Zugeinrichtung 9, wobei das proximale Ende des verschiebbaren Schneidenteils 8 und das distale Ende der Schub-/Zugeinrichtung 9 kraftschlüssig miteinander verbunden sind, um eine spielfreie Übertragung der Axialbewegung der Schub-/Zugeinrichtung 9 auf den verschiebbaren Schneidenteil 8 zu gewährleisten.

Wie weiterhin aus den Abbildungen 2 bis 6 ersichtlich, ist der starre Schneidenteil 7 über einen als Bajonettverbindung ausgebildeten Kopplungsmechanismus 11 lösbar am Schaft 2 festlegbar.

Fig. 2 zeigt eine Werkzeugspitze 6 gemäß dem Stand der Technik mit einem starren Schneidenteil 7 und einem verschiebbaren Schneidenteil 8, deren Schneidflächen 12 in einem Winkel von 45° zur Längsachse 10 des Schaftes 2 ausgebildet sind. Beim Überführen des verschiebbaren Schneidenteils 8 in die dargestellte geschlossene Stanzposition kommt es immer wieder vor, dass die Schneidenteile 7 und 8 aus ihrer achsparallelen Ausrichtung zueinander auseinanderscheren, wie dies in Fig. 2 dargestellt ist. In dieser gegeneinander verkippten Stellung der Schneidenteile 7 und 8 ist für den Operateur eine dosierte Betätigung der Stanzwerkzeuge nicht möglich.

Um die Schneidenteile 7 und 8 in ihrer achsparallelen Lage zueinander zu stabilisieren und deren Auseinanderscheren zu verhindern, ist an jeder der in den Abbildungen Fig. 3 bis 5 dargestellten Werkzeugspitzen 6 jeweils ein Kippschutz 13 angeordnet, der ein Verkippen der Schneidenteile 7 und 8 unmöglich macht.

Bei der in Fig. 3 dargestellten ersten Ausführungsform ist der Kippschutz 13 als im proximalen Bereich am verschiebbaren Schneidenteil I ausgebildetes Widerlager 14 in der Form eines Wulstes ausgebildet, an dem der starre Schneidenteil 7 anliegt und so die Achsparallelität der Schneidenteile 7 und 8 zueinander fixiert.

Bei der in Fig. 4 dargestellten zweiten Ausführungsform zur Ausgestaltung des Kippschutzes 13 ist der Kippschutz 13 als im distalen Bereich am starren Schneidenteil 7 angeordneter Führungsstift 15 ausgebildet, der in eine entsprechende Aufnahme 16 im verschiebbaren Schneidenteil 8 eingreift und so die Achsparallelität der Schneidenteile 7 und 8 zueinander in der geschlossenen Stanzposition fixiert.

Bei der in Fig. 5 dargestellten dritten Ausführungsform ist der Kippschutz 13 als die beiden Schneidenteile 7 und 8 im proximalen Bereich umgreifende Hülse 17 ausgebildet, die als äußerer Druckring die beiden Schneidenteile 7 und 8 gegeneinander fixiert.

Allen drei dargestellten Ausführungsformen ist gemeinsam, dass durch die Anordnung des Kippschutzes 13 an der Werkzeugspitze 6 das in Fig. 2 dargestellte, aus dem Stand der Technik bekannte Ausscheren der Schneidenteile 7 und 8 aus ihrer achsparallelen Anordnung zueinander verhindert wird, so dass für den Operateur immer ein exaktes und dosiertes Betätigen der Schneidenteile 7 und 8 der medizinischen Stanze 1 gewährleistet ist.

Wie weiterhin aus den Abbildungen Fig. 3 bis 6 ersichtlich, besteht der starre Schneidenteil 7 bei allen Ausführungsformen aus einem den verschiebbaren Schneidenteil 8 umgreifenden hülsenförmigen proximalen Teil 18 und einem einstückig mit dem hülsenförmigen Teil 18 ausgebildeten distalen Stanzteil 19, wobei im hülsenförmigen proximalen Teil 18 des starren Schneidenteils 7 eine Führungsbahn 20 zur führenden Aufnahme des verschiebbaren Schneidenteils 8 ausgebildet ist.

Zur Aufnahme und Führung des verschiebbaren Schneidenteils 8 ist die Führungsbahn 20 vorteilhafterweise so ausgebildet, dass sie die Außenkontur des aufzunehmenden verschiebbaren Schneidenteils 8 im Wesentlichen formschlüssig umschließt. Die lagegerechte Führung des verschiebbaren Schneidenteils 8 in der Führungsbahn 20 wird bei den in Fig. 6 und 7 dargestellten Ausführungsbeispielen dadurch stabilisiert, dass am verschiebbaren Schneidenteil 8 seitlich nach außen vorspringende Führungselemente 21 ausgebildet sind, die in eine korrespondierende Aufnahme 22 der Führungsbahn 20 eingreifen.

Die in Fig. 6 dargestellte Ausführungsform der Werkzeugspitze 6 unterscheidet sich von den in den Abbildungen Fig. 3 bis 5 dargestellten Ausführungsformen, durch die Ausgestaltung der Schneidflächen 12. Während bei der Ausführungsform gemäß Fig. 6 die Schneidflächen 12 der Schneidenteile 7 und 8 parallel zueinander und im Wesentlichen rechtwinklig zur Längsachse 10 des Schaftes 2 ausgebildet sind, sind die Schneidflächen 12 der Schneidenteile 7 und 8 der Ausführungsformen gemäß Fig. 3 bis 5 zwar ebenfalls parallel zueinander, jedoch in einem vom rechten Winkel abweichenden Winkel, vorzugsweise von 45°, zur Längsachse 10 des Schaftes 2 ausgerichtet ausgebildet.

Die Arbeitsweise und Funktionsweise der dargestellten und beschriebenen medizinischen Stanzen 1 ist jedoch völlig unabhängig von der Ausrichtung der Schneidflächen 12 zur Längsachse 10 des Schaftes 2.

Um die Montage und Demontage der Werkzeugspitze 6 mit den beiden Schneidteilen 7 und 8 zu erleichtern, sind, wie insbesondere aus Fig. 6 und 7 ersichtlich, der starre Schneidenteil 7 und der verschiebbare Schneidenteil 8 über eine auf den hülsenformigen Teil 18 des starren Schneidenteils 7 aufgeschobene hülsenförmige Manschette 23 zu einer Baueinheit miteinander verbunden.

Durch diese hülsenförmige Manschette 23 wird der starre Schneidenteil 7 unverlierbar an dem in der Führungsbahn 20 des starren Schneidenteils 7 gelagerten verschiebbaren Schneidenteil 8 festgelegt, wodurch eine Montageeinheit geschaffen wird, die über den am hülsenförmigen proximalen Teil 18 des starren Schneidenteils 7 angeordneten Kopplungsmechanismus 11 am hohlen Schaft 2 festlegbar ist.

Bei der in den Abbildungen Fig. 6 und 7 dargestellten Ausführungsform bildet die den verschiebbaren Schneidenteil 8 und den starren Schneidenteil 7 zu einer Baueinheit verbindende Manschette 23 gleichzeitig auch den die beiden Schneidenteile 7 und 8 gegeneinander stabilisierenden, als Hülse 17 ausgebildeten Kippschutz 13.

Wie weiterhin aus Fig. 6 ersichtlich, ist am starren Schneidenteil 7 ein die Verschiebbarkeit des verschiebbaren Schneidenteils 8 in die distale Richtung (offene Position) begrenzender Anschlag 24 ausgebildet.

Eine solchermaßen ausgebildete medizinische Stanze 1 zeichnet sich dadurch aus, dass sie bei einfachem konstruktivem Aufbau einerseits eine exakte und gut dosierbare Betätigung der Schneidenteile 7 und 8 ermöglicht und andererseits zu Montage- und Reinigungszwecken schnell und einfach zerlegbar und wieder zusammensetzbar ist.

### Bezuqszeichenliste

- 1: medizinische Stanze 18 hülsenförmiger Teil
- 2: Schaft 19 Stanzteil
- 3: Handhabe 20 Führungsbahn
- 4: starrer Griffteil 21 Führungselement
- 5: verschwenkbarer Griffteil 22 Aufnahme
- 6: Werkzeugspitze 23 Manschette
- 7: starrer Schneidenteil 24 Anschlag
- 8: verschiebbarer Schneidenteil
- 9: Schub-/Zugeinrichtung
- 10: Längsachse
- 11: Kopplungsmechanismus
- 12: Schneidfläche
- 13: Kippschutz
- 14: Widerlager
- 15: Führungsstift
- 16: Aufnahme
- 17: Hülse

## Patentansprüche

1. Medizinische Stanze mit einem hohlen Schaft (2), einer am distalen Ende des Schaftes (2) angeordneten, aus einem starren Schneidenteil (7) und einem gegenüber der starren Schneidteil (7) verschiebbaren Schneidteil (8) bestehenden Werkzeugspitze (6) sowie mit einer am proximalen Ende des Schaftes (2) angeordneten Handhabe (3), wobei der verschiebbare Schneidenteil (8) und die Handhabe (3) über eine im hohlen Schaft (2) verschiebbar gelagerte Schub-/Zugeinrichtung (9) miteinander in Wirkverbindung stehen und an der Werkzeugspitze (6) ein die Schneidenteile (7, 8) in ihrer zueinander achsparallelen Ausrichtung stabilisierender Kippschutz (13) angeordhet ist, wobei der starre Schneidenteil (7) aus einem den verschiebbaren Schneidenteil (8) umgreifenden hülsenförmigen proximalen Teil (18) und einem einstückig mit dem hülsenförmigen Teil (18) ausgebildeten distalen Stanzteil (19) besteht,
**dadurch gekennzeichnet,**
**dass** der starre Schneidenteil (7) und der verschiebbare Schneidenteil (8) über eine auf den hülsenförmigen Teil (18) des starren Schneidenteils (7) aufschiebbare hülsenförmige Manschette (23) zu einer Baueinheit verbindbar sind und, dass diese Baueinheit über einen am hülsenförmigen Teil (18) des starren Schneidenteils (7) ausgebildeten Kopplungsmechanismus (11) lösbar mit dem hohlen Schaft (2) verbunden ist.

2. Medizinische Stanze nach Anspruch 1, **dadurch gekennzeichnet, dass** in dem hülsenförmigen Teil (18) des starren Schneidenteils (7) eine Führungsbahn (20) zur Aufnahme des verschiebbaren Schneidenteils (8) ausgebildet ist.

3. Medizinische Stanze nach Anspruch 2, **dadurch gekennzeichnet, dass** am verschiebbaren Schneidenteil (8) mindestens den verschiebbaren Schneidenteil (8) in der Führungsbahn (20) stabiiisierendes Führungselement (21) ausgebildet ist.

4. Medizinische Stanze nach einem der Ansprüche 1 bis 3, **dadurch gekennzeichnet, dass** der Kippschutz (13) als an einem Schneidenteil (7 oder 8) im proximalen Bereich ausgebildetes Widerlager (14) ausgebildet ist, an dem der andere Schneidenteil (8 oder 7) anliegt.

5. Medizinische Stanze nach einem der Ansprüche 1 bis 3, **dadurch gekennzeichnet, dass** der Kippschutz (13) als an einem Schneidenteil (7 oder 8) im distalen Bereich ausgebildeter Führungsstift (15) ausgebildet ist, der in eine entsprechende Aufnahme (16) im anderen Schneidenteil (8 oder 7) eingreift.

6. Medizinische Stanze nach einem der Ansprüche 1 bis 3, **dadurch gekennzeichnet, dass** der Kippschutz (13) als beide Schneidenteile (7, 8) im proximalen Bereich umgreifende Hülse (17) ausgebindet ist.

7. Medizinische Stanze nach einem der Ansprüche 1 bis 6, **dadurch gekennzeichnet, dass** der verschlebbare Schneidenteil (8) zumindest kraftschlüssig mit der Schub-/Zugeinrichtung (9) verbunden ist.

8. Medizinische Stanze nach einem der Ansprüche 1 bis 7, **dadurch gekennzeichnet, dass** die Schub-/Zugeinrichtung (9) über das distale Ende des Schaftes (2) in den Schaft (2) einsetzbar und aus dem Schaft (2) herausziehbar ist.

9. Medizinische Stanze nach Anspruch 6, **dadurch gekennzeichnet, dass** die hülsenförmige Manschette (23) gleichzeitig die den Kippschutz (13) bildende, beide Schneidenteile (7, 8) im proximalen Bereich umgreifende Hülse (17) ist.

10. Medizinische Stanze nach einem der Ansprüche 1 bis 9, **dadurch gekennzeichnet, dass** am starren Schneidenteil (7) die Verschiebbarkeit des verschiebbaren Schneidenteils (8) in die distale Richtung begrenzender Anschlag (24) ausgebildet ist.

## Claims

1. Medical punch having a hollow shaft (2), a tool tip (6) situated at the distal end of the shaft (2) and composed of a fixed cutting part (7) and a cutting part (8) which is displaceable with respect to the fixed cutting part (7), and a handle (3) situated at the proximal end of the shaft (2), wherein the displaceable cutting part (8) and the handle (3) are in operative connection with one another via a push/pull device (9) displaceably supported in the hollow shaft (2), and that an anti-tilt protector (13) which stabilizes the cutting parts (7, 8) in their mutually axially parallel orientation is situated at the tool tip (6), wherein the fixed cutting part (7) is composed of a sleeve-shaped proximal portion (18) which encloses the displaceable cutting part (8), and a distal punching part (19) which is formed in one piece with the sleeve-shaped portion (18),
**characterized in that** the fixed cutting part (7) and the displaceable cutting part (8) are connectable via a sleeve-shaped collar (23) which may be pushed onto the sleeve-shaped portion (18) of the fixed cutting part (7) to form an integral unit, and that this integral unit is detachably connected to the hollow shaft (2) via a coupling mechanism (11) provided on the sleeve-shaped portion (18) of the fixed cutting part (7).

2. Medical punch according to Claim 1, **characterized in that** a guideway (20) for accommodating the displaceable cutting part (8) is formed in the sleeve-shaped portion (18) of the fixed cutting part (7).

3. Medical punch according to Claim 2, **characterized in that** at least the guide element (21) which stabilizes the displaceable cutting part (8) in the guideway (20) is provided at the displaceable cutting part (8).

4. Medical punch according to one of Claims 1 through 3, **characterized in that** the anti-tilt protector (13) is designed as an abutment (14), formed at a cutting part (7 or 8) in the proximal area, against which the other respective cutting part (8 or 7) abuts.

5. Medical punch according to one of Claims 1 through 3, **characterized in that** the anti-tilt protector (13) is designed as a guide pin (15) which is provided at a cutting part (7 or 8) in the distal area, and which engages with a corresponding receptacle (16) in the other respective cutting part (8 or 7).

6. Medical punch according to one of Claims 1 through 3, **characterized in that** the anti-tilt protector (13) is designed as a sleeve (17) which encloses both cutting parts (7, 8) in the proximal area.

7. Medical punch according to one of Claims 1 through 6, **characterized in that** the displaceable cutting part (8) is connected in at least a positive-fit manner to the push/pull device (9).

8. Medical punch according to one of Claims 1 through 7, **characterized in that** the push/pull device (9) is insertable into the shaft (2) and retractable from the shaft (2) via the distal end of the shaft (2).

9. Medical punch according to Claim 6, **characterized in that** the sleeve-shaped collar (23) at the same time is the sleeve (17) which forms the anti-tilt protector (13) and encloses both cutting parts (7, 8) in the proximal area.

10. Medical punch according to one of Claims 1 through 9, **characterized in that** [a] stop (24) which limits the displaceability of the displaceable cutting part (8) in the distal direction is provided at the fixed cutting part (7).

## Revendications

1. Instrument médical de coupe par poinçonnage comprenant un corps allongé creux (2), une pointe d'outil (6) agencée à l'extrémité distale du corps allongé (2) et constituée d'une pièce de coupe fixe (7) et d'une pièce de coupe (8) pouvant coulisser par rapport à la pièce de coupe fixe (7), et comprenant également une poignée de manoeuvre (3) agencée à l'extrémité proximale du corps allongé (2), instrument médical
dans lequel la pièce de coupe coulissante (8) et la poignée de manoeuvre (3) sont en liaison d'interaction par l'intermédiaire d'un dispositif de poussée/traction (9) monté coulissant dans le corps allongé creux (2), dans lequel au niveau de la pointe d'outil (6) est agencé un système de sécurité au basculement (13) stabilisant les pièces de coupe (7, 8) dans leur orientation à axes parallèles, et
dans lequel la pièce de coupe fixe (7) est constituée d'une partie proximale (18) en forme de fourreau entourant la pièce de coupe coulissante (8), et d'une partie de poinçonnage distale (19) réalisée d'un seul tenant avec la partie en forme de fourreau (18),
**caractérisé en ce que** la pièce de coupe fixe (7) et la pièce de coupe coulissante (8) peuvent être reliées en un module de construction par l'intermédiaire d'une manchette (23) en forme de fourreau pouvant être engagée par coulissement par-dessus la partie (18) en forme de fourreau de la pièce de coupe fixe (7), et **en ce que** ce module de construction est relié au corps allongé creux (2), par l'intermédiaire d'un mécanisme de couplage (11) réalisé sur la partie (18) en forme de fourreau de la pièce de coupe fixe (7).

2. Instrument médical de coupe par poinçonnage selon la revendication 1,
**caractérisé en ce que** dans la partie (18) en forme de fourreau de la pièce de coupe fixe (7) est réalisée une voie de guidage (20) destinée à recevoir la pièce de coupe coulissante (8).

3. Instrument médical de coupe par poinçonnage selon la revendication 2,
**caractérisé en ce que** sur la pièce de coupe coulissante (8) est réalisé un élément de guidage (21) stabilisant au moins la pièce de coupe coulissante (8) dans la voie de guidage (20).

4. Instrument médical de coupe par poinçonnage selon l'une des revendications 1 à 3,
**caractérisé en ce que** le système de sécurité au basculement (13) est réalisé en tant que butée d'appui (14) formée sur une des pièces de coupe (7 ou 8), dans la zone proximale, et contre laquelle vient s'appuyer l'autre pièce de coupe (8 ou 7).

5. Instrument médical de coupe par poinçonnage selon l'une des revendications 1 à 3,
**caractérisé en ce que** le système de sécurité au basculement (13) est réalisé en tant que broche de guidage (15), qui est formée sur une des pièces de coupe (7 ou 8), dans la zone distale, et s'engage dans un logement de réception (16) correspondant dans l'autre pièce de coupe (8 ou 7).

6. Instrument médical de coupe par poinçonnage selon l'une des revendications 1 à 3,
**caractérisé en ce que** le système de sécurité au basculement (13) est réalisé en tant que fourreau (17) entourant les deux pièces de coupe (7, 8) dans la zone proximale.

7. Instrument médical de coupe par poinçonnage selon l'une des revendications 1 à 6,
**caractérisé en ce que** la pièce de coupe coulissante (8) est reliée au moins par une liaison par adhérence, au dispositif de poussée/traction (9).

8. Instrument médical de coupe par poinçonnage selon l'une des revendications 1 à 7,
**caractérisé en ce que** le dispositif de poussée/traction (9) peut être inséré dans le corps allongé (2) et retiré du corps allongé (2), à travers l'extrémité distale du corps allongé (2).

9. Instrument médical de coupe par poinçonnage selon la revendication 6,
**caractérisé en ce que** la manchette (23) en forme de fourreau est simultanément le fourreau (17) formant le système de sécurité au basculement (13) entourant les deux pièces de coupe (7, 8) dans la zone proximale.

10. Instrument médical de coupe par poinçonnage selon l'une des revendications 1 à 9,
**caractérisé en ce que** sur la pièce de coupe fixe (7) est réalisée une butée (24) limitant la possibilité de coulissement de la pièce de coupe coulissante (8) dans la direction distale.
